# EUROPEAN PATENT APPLICATION

(11) **EP 2 060 257 A1**
(43) Date of publication of application: **20.05.2009**
(21) Application number: 07120259.2
(22) Date of filing: 08.11.2007
(51) Int. Cl.: A61K 31/35, A61K 31/702, A61K 31/726, C07D 309/10, C07D 309/14, A61P 31/00, A61P 31/04

(54) **Prevention and treatment of secondary infections following viral infection**

(71) Applicant: Nestec S.A., 1800 Vevey (CH)
(72) Inventor: Fichot, Marie-Claire, 1807 Blonay (CH); Sprenger, Norbert, 1073 Savigny (CH)
(74) Representative: Dixon, Sarah

(57) **Abstract**

This invention relates to a composition suitable for use in the prevention of secondary infections following a viral infection characterised by neuraminidase activity comprising a sialylated oligosaccharide and N-acetyl-lactosamine and/or an oligosaccharide containing N-acetyl-lactosamine The invention further extends to the use of such a composition in the prevention of secondary infections such as otitis media.

## Description

### Field of the Invention

This invention relates to the prevention and treatment of secondary infections following viral infection, particularly in infants and small children.

### Background of the Invention

Infections of the respiratory tract are very common, particularly in infants and small children. For example, in the first year of life, an infant will often experience from three to six such infections. Such infections are usually of bacterial origin and often follow a viral infection such as influenza. Examples of bacterial infections of the respiratory tract include pneumonia, sinusitis and otitis media.

Frequent respiratory tract infections are often associated with acute otitis media. This is an infection of the middle ear in which the Eustachian tube which connects the cavity of the middle ear with the external environment via the mouth becomes inflamed and then blocked trapping bacteria in the middle ear. The middle ear cavity also becomes inflamed with a build up of fluid leading to increased pressure which is experienced by the patient as pain due to the inability to equalise pressure between the middle ear and the external environment via the Eustachian tube as in healthy subjects. In severe cases, the tympanic membrane may burst under pressure allowing the infected liquid to reach the inner ear. This is a potentially dangerous situation which can lead to permanently impaired hearing if left untreated.

50% of children will have had at least one episode of acute otitis media in the first year of life and 35% of children between one and three years of age have recurrent episodes of acute otitis media. This in turn may lead to the development of a condition called glue ear in which the fluid does not completely drain from the middle ear between bouts of infection. If this condition becomes established, surgical intervention may be necessary.

Acute otitis media appears to be linked with the activity of pathogenic bacteria commonly found in the indigenous microbiota of the naso-pharyngeal cavity. Quantitatively, the most important pathogens are *Streptococcus pneumoniae* (35% of cases), untypeable *Haemophilus influenzae* (30% of cases) and *Moraxella catarrhalis* (10% of cases). For this reason, acute otitis media is commonly treated by the administration of antibiotics especially in infants. Indeed, antibiotics are prescribed more frequently for treatment of otitis media than for any other illness in infancy. This has inevitably led to the development of resistance to the commonly prescribed antibiotics in the bacterial strains associated with otitis media. For example, it is thought that at least 20% of *S. pneumoniae* strains are resistant to penicillins and cephalosporins. Similarly, at least 30% of *H. influenzae* strains and the majority of *M. catarrhalis* strains have developed antibiotic resistance. This frequency of prescription is at least in part due to the pain experienced by infants and young children suffering from otitis media to which they react by prolonged crying which parents and other care givers are very anxious to relieve. There is thus clearly a need for alternative methods to decrease the incidence of this painful and potentially serious condition in infants and young children.

Various alternative therapies have already been proposed. For example, in WO 97/17089 it is proposed to use a so-called immune milk preparation for the prevention of otitis media. This preparation contains anti-otitis immunoglobulins of the IgG type obtained from bovine colostrum to complement the passive immune defence.

Various bacterial strains have also been proposed for prevention/treatment of otitis media. In a recent clinical trial, inhibitory alpha haemolytic streptococci were sprayed into the noses of children with acute otitis media. The strains used were *Streptococcus mitis, Streptococcus sanguis* and *Streptococcus oralis.* The children treated in this way had less episodes of acute otitis media (Roos et al, Effect of recolonisation with "interfering alpha streptococci" on recurrences of acute and secretory otitis media in children: randomised placebo controlled trial, BMJ 322:210-212). However, the bacterial strains used in this trial are also recognised human pathogens implicated in conditions such as endocarditis and lung infections.

WO 2004/072272 proposes the use of a specific strain of *Streptococcus salivarius* in the prevention and treatment of otitis media. This strain is stated to be a bacteriocin producing strain which is non-pathogenic. It may be administered intranasally, by inhalation via the mouth or in the form of lozenges or capsules. Preferably, the strain is administered after an initial treatment with an antibiotic or other anti-microbial agent.

Likewise, bacterial infections may also follow infection with other viruses.

From the foregoing, it may be seen that there is a need for an effective method for the prevention of secondary infections following viral infections such as influenza which does not rely on the use of antibiotics and which may be conveniently and safely administered.

### Summary of the Invention

The present inventors have surprisingly found that the co-administration of sialylated oligosaccharides and N-acetyl-lactosamine and/or oligosaccharides containing N-acetyl-lactosamine is particularly effective in the prevention of secondary infections following viral infections such as influenza.

Accordingly, in a first aspect, the present invention provides a composition suitable for use in the prevention of secondary infections following a viral infection characterised by neuraminidase activity comprising a sialylated oligosaccharide and N-acetyl-lactosamine and/or an oligosaccharide containing N-acetyl-lactosamine.

In a second aspect, the present invention provides the use of a sialylated oligosaccharide and N-acetyl-lactosamine and/or an oligosaccharide containing N-acetyl-lactosamine in the manufacture of a composition for the prevention of secondary infections following a viral infection characterised by neuraminidase activity.

The invention further extends to a method for the prevention of secondary infections following a viral infection characterised by neuraminidase activity which comprises administering to an individual in need thereof a therapeutic amount of a sialylated oligosaccharide and N-acetyl-lactosamine and/or an oligosaccharide containing N-acetyl-lactosamine.

Without wishing to be bound by theory, the inventors believe that the efficacy of the combination of oligosaccharides described above in the prevention of secondary infections following influenza for example may be a result of disruption of the synergy between the actions of the viral and bacterial pathogens. Specifically, it is known that successful replication of the influenza virus in host epithelial cells relies upon the action of neuraminidases on the surface of the viral particles to free the newly replicated viral particles from the host cell by cleaving the sialic acid residues that bind the particles to the host cell. Indeed, the medicines most commonly prescribed for influenza are neuraminidase inhibitors. Further, it is thought that it is the resulting desialylated epithelial cell surfaces that are particularly vulnerable to adhesion of pathogenic bacteria resulting in secondary infection. By supplying excess sialylated oligosaccharides, the efficiency of the viral neuraminidases can be reduced thus reducing the proportion of desialylated epithelial cell surfaces whilst at the same time neutral oligosaccharides which mimic the preferred epithelial binding sites of pathogenic bacteria are supplied in excess.

### Detailed Description of the Invention

In this specification, the following terms have the following meanings:-
"infant" means a child under the age of 12 months;
"infant formula" means a foodstuff intended for particular nutritional use by infants during the first four to six months of life and satisfying by itself the nutritional requirements of this category of person;
"follow-on formula" means a foodstuff intended for particular nutritional use by infants aged over four months and constituting the principal liquid element in the progressively diversified diet of this category of person;
"growing up milk" means a milk based beverage adapted for the specific nutritional needs of young children;
"pathogenic bacterial strain" means a bacterial strain which is a recognised human pathogen;
"prevention of secondary infection following a viral infection" means prevention or reduction of risk of or reduction of severity of a secondary infection by pathogenic bacteria following a viral infection;
"young child" means a child between the age of one and six years.

All percentages are by weight unless otherwise stated.

Suitable sialylated oligosaccharides include 3' sialyllactose and 6' sialyllactose. Preferably both 3' sialyllactose and 6' sialyllactose are present. 3'sialyl-lactose and 6'sialyl-lactose may be isolated by chromatographic or filtration technology from a natural source such as animal milks. Alternatively, they may be produced by biotechnology using specific sialyltransferases either by enzyme based fermentation technology (recombinant or natural enzymes) or by microbial fermentation technology. In the latter case microbes may either express their natural enzymes and substrates or may be engineered to produce respective substrates and enzymes. Single microbial cultures or mixed cultures may be used. Sialyl-oligosaccharide formation can be initiated by acceptor substrates starting from any degree of polymerisation (DP) from DP=1 onwards. Alternatively, sialyllactoses may be produced by chemical synthesis starting with lactose and free N'acetylneuraminic acid (sialic acid). Sialyllactoses are also commercially available for example from Kyowa Hakko Kogyo of Japan.

In addition to a sialylated oligosaccharide, the composition according to the invention also contains N-acetyl-lactosamine and/or an oligosaccharide containing N-acetyl-lactosamine. Suitable oligosaccharides containing N-acetyl-lactosamine include lacto-N-tetraose (LNT) and lacto-N-neotetraose (LNnT). LNT and LNnT may be synthesised chemically by enzymatic transfer of saccharide units from donor moieties to acceptor moieties using glycosyltransferases as described for example in US Patent No. 5,288,637. Alternatively, LNT and LNnT may be prepared by chemical conversion of keto-hexoses (e.g. fructose) either free or bound to an oligosaccharide (e.g. lactulose) into N-acetylhexosamine or an N-acetylhexosamine containing oligosaccharide as described in Wrodnigg, T.M.; Stutz, A.E. (1999) Angew. Chem. Int. Ed. 38:827-828. N-acetyllactosamine produced in this way may then be transferred to lactose as acceptor moiety.

The oligosaccharides may be administered in the same composition or may be administered sequentially.

The secondary infections which may be prevented according to the invention include infections of the respiratory tract such as pneumonia, sinusitis and otitis media as well as infections of the gastrointestinal tract. The invention is particularly suitable for the prevention of secondary infections of the respiratory tract such as otitis media after influenza in infants and young children.

If this age group is to be addressed, the composition is preferably a nutritional composition which is consumed as a liquid. It may be a nutritionally complete formula such as an infant formula, a follow-on formula or a growing up milk. Alternatively for the older end of the target group of infants and young children, the composition may be a juice drink or other chilled or shelf stable beverage or a soup, for example.

Preferably a nutritional composition according to the invention contains from 0.1 to 2 g of sialylated oligosaccharides /100g nutritional composition on a dry weight basis and from 0.1 to 3g N-acetyl-lactosamine or oligosaccharide containing N-acetyl-lactosamine lactose/100g nutritional composition on a dry weight basis.

If the composition contains both 3' sialyllactose and 6' sialyllactose, these two compounds are preferably present in a ratio between 5:1 and 1:2 (3' sialyllactose:6' sialyllactose).

A nutritional composition according to the invention preferably further contains at least one prebiotic in an amount of 0.3 to 10%. A prebiotic is a non-digestible food ingredient that beneficially affects the host by selectively stimulating the growth and/or activity of one or a limited number of bacteria in the colon, and thus improves host health. Such ingredients are non-digestible in the sense that they are not broken down and absorbed in the stomach or small intestine and thus pass intact to the colon where they are selectively fermented by the beneficial bacteria. Examples of prebiotics include certain oligosaccharides, such as fructooligosaccharides (FOS) and galactooligosaccharides (GOS). A combination of prebiotics may be used such as 90% GOS with 10% short chain fructo-oligosaccharides such as the product sold under the trade mark Raftilose® or 10% inulin such as the product sold under the trade mark Raftiline®. A particularly preferred combination of prebiotics is 70% short chain fructo-oligosaccharides and 30% inulin.

A composition according to the invention may also comprise at least one probiotic bacterial strain. A probiotic is a microbial cell preparation or components of microbial cells with a beneficial effect on the health or well-being of the host. Suitable probiotic bacterial strains include *Lactobacillus rhamnosus* ATCC 53103 obtainable from Valio Oy of Finland under the trade mark LGG, *Lactobacillus rhamnosus* CGMCC 1.3724, *Lactobacillus paracasei* CNCM I-2116, the strain of *Lactobacillus reuteri* sold by BioGaia A.B under the trade mark Reuteri, *Streptococcus salivarius* DSM 13084 sold by BLIS Technologies Limited of New Zealand under the designation K12, *Bifidobacterium lactis* CNCM 1-3446 sold *inter alia* by the Christian Hansen company of Denmark under the trade mark Bb12, *Bifidobacterium longum* ATCC BAA-999 sold by Morinaga Milk Industry Co. Ltd. of Japan under the trade mark BB536, the strain of *Bifidobacterium breve* sold by Danisco under the trade mark Bb-03, the strain of *Bifidobacterium breve* sold by Morinaga under the trade mark M-16V, the strain of Bifidobacterium infantis sold by Procter & Gamble Co. under the trade mark Bifantis and the strain of *Bifidobacterium breve* sold by Institut Rosell (Lallemand) under the trade mark R0070 in an amount between 10e3 and 10e12 cfu/g powder, more preferably between 10e7 and 10e12 cfu/g powder

The general composition of an infant formula according to the invention will now be described by way of example. The formula contains a protein source.

The type of protein is not believed to be critical to the present invention provided that the minimum requirements for essential amino acid content are met and satisfactory growth is ensured. Thus, protein sources based on whey, casein and mixtures thereof may be used as well as protein sources based on soy. As far as whey proteins are concerned, the protein source may be based on acid whey or sweet whey or mixtures thereof and may include alpha-lactalbumin and betalactoglobulin in whatever proportions are desired.

The proteins may be intact or hydrolysed or a mixture of intact and hydrolysed proteins. It may be desirable to supply partially hydrolysed proteins (degree of hydrolysis between 2 and 20%), for example for infants believed to be at risk of developing cows' milk allergy. If hydrolysed proteins are required, the hydrolysis process may be carried out as desired and as is known in the art. For example, a whey protein hydrolysate may be prepared by enzymatically hydrolysing the whey fraction in one or more steps. If the whey fraction used as the starting material is substantially lactose free, it is found that the protein suffers much less lysine blockage during the hydrolysis process. This enables the extent of lysine blockage to be reduced from about 15% by weight of total lysine to less than about 10% by weight of lysine; for example about 7% by weight of lysine which greatly improves the nutritional quality of the protein source.

An infant formula according to the present invention contains a carbohydrate source. Any carbohydrate source conventionally found in infant formulae such as lactose, saccharose, maltodextrin, starch and mixtures thereof may be used although the preferred source of carbohydrates is lactose. Preferably the carbohydrate sources contribute between 35 and 65% of the total energy of the formula.

An infant formula according to the present invention contains a source of lipids. The lipid source may be any lipid or fat which is suitable for use in infant formulas. Preferred fat sources include palm olein, high oleic sunflower oil and high oleic safflower oil. The essential fatty acids linoleic and α-linolenic acid may also be added as may small amounts of oils containing high quantities of preformed arachidonic acid and docosahexaenoic acid such as fish oils or microbial oils. In total, the fat content is preferably such as to contribute between 30 to 55% of the total energy of the formula. The fat source preferably has a ratio of n-6 to n-3 fatty acids of about 5:1 to about 15:1; for example about 8:1 to about 10:1.

The infant formula will also contain all vitamins and minerals understood to be essential in the daily diet and in nutritionally significant amounts. Minimum requirements have been established for certain vitamins and minerals. Examples of minerals, vitamins and other nutrients optionally present in the infant formula include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chloride, potassium, sodium, selenium, chromium, molybdenum, taurine, and L-carnitine. Minerals are usually added in salt form. The presence and amounts of specific minerals and other vitamins will vary depending on the intended infant population.

If necessary, the infant formula may contain emulsifiers and stabilisers such as soy lecithin, citric acid esters of mono- and di-glycerides, and the like.

The infant formula may optionally contain other substances which may have a beneficial effect such as lactoferrin, nucleotides, nucleosides, and the like.

Finally, the formula will contain 3' sialyllactose and 6' sialyllactose in a total amount between 0.1 to 2 g of sialyllactoses /100g formula and LNnT in an amount between 0.1 to 3g LNnT/100g formula. The ratio 3' sialyllactose:6' sialyllactose will be between 5:1 and 1:2.

The formula may be prepared in any suitable manner. For example, it may be prepared by blending together the protein, the carbohydrate source, and the fat source in appropriate proportions. If used, the emulsifiers may be included at this point. The vitamins and minerals may be added at this point but are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture. The temperature of the water is conveniently about 50°C to about 80°C to aid dispersal of the ingredients. Commercially available liquefiers may be used to form the liquid mixture. The sialylated oligosaccharides and N-acetyl-lactosamine and/or an oligosaccharide containing N-acetyl-lactosamine will be added at this stage if the final product will be liquid form. If the final product is to be a powder, the oligosaccharides may likewise be added at this stage if desired. The liquid mixture is then homogenised; for example in two stages.

The liquid mixture may then be thermally treated to reduce bacterial loads, by rapidly heating the liquid mixture to a temperature in the range of about 80°C to about 150°C for about 5 seconds to about 5 minutes, for example. This may be carried out by steam injection, autoclave or by heat exchanger; for example a plate heat exchanger.

Then, the liquid mixture may be cooled to about 60°C to about 85°C; for example by flash cooling. The liquid mixture may then be again homogenised; for example in two stages at about 10 MPa to about 30 MPa in the first stage and about 2 MPa to about 10 MPa in the second stage. The homogenised mixture may then be further cooled to add any heat sensitive components; such as vitamins and minerals. The pH and solids content of the homogenised mixture are conveniently adjusted at this point.

The homogenised mixture is transferred to a suitable drying apparatus such as a spray drier or freeze drier and converted to powder. The powder should have a moisture content of less than about 5% by weight. The siallylated oligosaccharides and N-acetyl-lactosamine and/or an oligosaccharide containing N-acetyl-lactosamine may be added at this stage by dry-mixing along with the probiotic bacterial strain(s) if used.

If a liquid product is preferred, the homogenised mixture may be sterilised then aseptically filled into suitable containers or may be first filled into the containers and then retorted.

In another embodiment, the composition may be a supplement including the sialylated oligosaccharide and N-acetyl-lactosamine and/or an oligosaccharide containing N-acetyl-lactosamine in an amount sufficient to achieve the desired effect in an individual. This form of administration is more suited to older children and adults. Preferably the daily dose of the sialylated oligosaccharide is from 0.1 to 2g and the daily dose of the N-acetyl-lactosamine and/or an oligosaccharide containing N-acetyl-lactosamine is from 0.1 to 3g. The amount of oligosaccharides to be included in the supplement will be selected accordingly depending upon how the supplement is to be administered. For example, if the supplement is to be administered twice a day, each supplement may contain 0.05 to 1g sialylated oligosaccharide and 0.05 to 1.5g N-acetyl-lactosamine and/or an oligosaccharide containing N-acetyl-lactosamine. The supplement may be in the form of tablets, capsules, pastilles or a liquid for example. The supplement may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents and gel forming agents. The supplement may also contain conventional pharmaceutical additives and adjuvants, excipients and diluents, including, but not limited to, water, gelatine of any origin, vegetable gums, ligninsulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like.

Further, the supplement may contain an organic or inorganic carrier material suitable for oral or enteral administration as well as vitamins, minerals trace elements and other micronutrients in accordance with the recommendations of Government bodies such as the USRDA.

### Example 1

An example of the composition of an infant formula according to the present invention is given below. This composition is given by way of illustration only.

| Nutrient | per 100kcal | per litre |
|---|---|---|
| Energy (kcal) | 100 | 670 |
| Protein (g) | 1.83 | 12.3 |
| Fat (g) | 5.3 | 35.7 |
| Linoleic acid (g) | 0.79 | 5.3 |
| α-Linolenic acid (mg) | 101 | 675 |
| Lactose (g) | 11.2 | 74.7 |
| Prebiotic (70%FOS, 30% inulin) (g) | 0.64 | 4.3 |
| Minerals (g) | 0.37 | 2.5 |
| Na (mg) | 23 | 150 |
| K (mg) | 89 | 590 |
| Cl (mg) | 64 | 430 |
| Ca (mg) | 62 | 410 |
| P (mg) | 31 | 210 |
| Mg (mg) | 7 | 50 |
| Mn (µg) | 8 | 50 |
| Se (µg) | 2 | 13 |
| Vitamin A (µg RE) | 105 | 700 |
| Vitamin D (µg) | 1.5 | 10 |
| Vitamin E (mg TE) | 0.8 | 5.4 |
| Vitamin K1 (µg) | 8 | 54 |
| Vitamin C (mg) | 10 | 67 |
| Vitamin B1 (mg) | 0.07 | 0.47 |
| Vitamin B2 (mg) | 0.15 | 1.0 |
| Niacin (mg) | 1 | 6.7 |
| Vitamin B6 (mg) | 0.075 | 0.50 |
| Folic acid (µg) | 9 | 60 |
| Pantothenic acid (mg) | 0.45 | 3 |
| Vitamin B12 (µg) | 0.3 | 2 |
| Biotin (µg) | 2.2 | 15 |
| Choline (mg) | 10 | 67 |
| Fe (mg) | 1.2 | 8 |
| I (µg) | 15 | 100 |
| Cu (mg) | 0.06 | 0.4 |
| Zn (mg) | 0.75 | 5 |
| 3' sialyllactose (mg) | 30 | 200 |
| 6' sialyllactose (mg) | 6 | 40 |
| LNnT (mg) | 30 | 200 |

## Claims

1. A composition suitable for use in the prevention of secondary infections following a viral infection characerised by neuraminidase activity comprising a sialylated oligosaccharide and N-acetyl-lactosamine and/or an oligosaccharide containing N-acetyl-lactosamine.

2. A composition according to Claim 1 wherein the sialylated oligosaccharide is selected from the group comprising 3' sialyllactose and 6' sialyllactose.

3. A composition according to Claim 2 which comprises both 3' sialyllactose and 6' sialyllactose.

4. A composition according to Claim 3, wherein the ratio between 3' sialyllactose and 6' sialyllactose is between 5:1 and 1:2.

5. A composition according to any preceding claim wherein the oligosaccharide containing N-acetyl-lactosamine is selected from the group comprising lacto-N- tetraose and lacto-N-neotetraose.

6. A composition according to any preceding claim which further comprises a probiotic bacterial strain.

7. A composition according to any preceding claim which is an infant formula, a follow-on formula or a growing-up milk.

8. A composition according to Claim 7 which comprises from 0.1 to 2g sialylated oligosaccharide/100g composition.

9. A composition according to Claim 7 or 8 which comprises from 0.1 to 3g N-acetyl-lactosamine and/or an oligosaccharide containing N-acetyl-lactosamine/100g composition.

10. A composition according to any of Claims 7 to 9 which further comprises at least one prebiotic in an amount of from 0.3 to 10% by weight of the composition.

11. A composition according to any of Claims 1 to 6 which is a supplement and which comprises from 0.1 to 2g sialylated oligosaccharide and 0.1 to 3g N-acetyl-lactosamine and/or an oligosaccharide containing N-acetyl-lactosamine per unit dose.

12. Use of a composition as claimed in any one of claims 1 to 11 in the manufacture of a medicament or therapeutic nutritional composition for the prevention of secondary infection following a viral infection **characterized by** neuraminidase activity.

13. The use of Claim 12 wherein the viral infection **characterized by** neuraminidase activity is influenza.

14. The use of Claim 13 wherein the secondary infection is an infection of the respiratory tract.

15. The use of Claim 12 or 13 wherein the secondary infection is otitis media.
